(19) 

Europäisches
Patentamt
European
Patent Office
Office européen
des brevets

(11) **EP 4 035 671 A1**

(12) **EUROPEAN PATENT APPLICATION**
published in accordance with Art. 153(4) EPC

(43) Date of publication:
**03.08.2022 Bulletin 2022/31**

(21) Application number: **20868679.0**

(22) Date of filing: **04.08.2020**

(51) International Patent Classification (IPC):
*A61K 38/17* (2006.01)          *A61K 35/20* (2006.01)
*A61K 38/07* (2006.01)          *A61P 3/00* (2006.01)
*A61P 3/04* (2006.01)          *A61P 25/02* (2006.01)
*A61P 25/26* (2006.01)          *C07K 5/103* (2006.01)
*C07K 5/107* (2006.01)          *C07K 14/47* (2006.01)

(52) Cooperative Patent Classification (CPC):
**A61K 35/20; A61K 38/07; A61K 38/17; A61P 3/00;
A61P 3/04; A61P 25/02; A61P 25/26; C07K 14/47**

(86) International application number:
**PCT/JP2020/029893**

(87) International publication number:
**WO 2021/059755 (01.04.2021 Gazette 2021/13)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **26.09.2019 JP 2019175693**

(71) Applicant: **Morinaga Milk Industry Co., Ltd.
Minato-ku
Tokyo 108-8384 (JP)**

(72) Inventors:
• **YAGO, Takumi
Zama-shi, Kanagawa 252-8583 (JP)**
• **SAKATA, Yasuyuki
Zama-shi, Kanagawa 252-8583 (JP)**
• **SAITO, Noriko
Zama-shi, Kanagawa 252-8583 (JP)**

(74) Representative: **Hoffmann Eitle
Patent- und Rechtsanwälte PartmbB
Arabellastraße 30
81925 München (DE)**

(54) **COMPOSITION FOR SYMPATHETIC NERVE ACTIVATION**

(57) An object of the present invention is to provide a technique capable of activating sympathetic nerve. A composition for sympathetic nerve activation, comprising one or more peptides selected from the group consisting of peptides consisting of any of amino acid sequences shown below (SEQ ID NOs: 1 to 16) and peptides including the amino acid sequences.
(Amino acid sequences: VAPFPE, HLPLP, WPPF, HQPHQPLPPT, TEDELQDK, LNPWDQ, IISQE, ANEEEY, VIPY, LPLP, VIESPPEIN, FPQY, LNVPGE, IVPNSAEE, GEPTSTPT, KFQSEEQQQ)

Energy expenditure (relative value with respect to water-administered group) (0-60 minutes)

*FIG. 1*

EP 4 035 671 A1

SEQUENCE LISTING

<110>  Morinaga milk industry co., ltd.

<120>  Composition for sympathetic nerve activation

<130>  FP341-201009

<150>  JP2019-175693
<151>  2019-09-26

<160>  16

<170>  PatentIn version 3.5

<210>  1
<211>  6
<212>  PRT
<213>  Artificial Sequence

<220>
<223>  pep1

<400>  1

Val Ala Pro Phe Pro Glu
1               5


<210>  2
<211>  5
<212>  PRT
<213>  Artificial Sequence

<220>
<223>  pep2

<400>  2

His Leu Pro Leu Pro
1               5


<210>  3
<211>  5
<212>  PRT
<213>  Artificial Sequence

<220>
<223>  pep3

<400>  3

Val Val Pro Pro Phe
1               5


<210>  4
<211>  10
<212>  PRT
<213>  Artificial Sequence

<220>

```
<223>  pep4

<400>  4

His Gln Pro His Gln Pro Leu Pro Pro Thr
1               5                   10


<210>  5
<211>  8
<212>  PRT
<213>  Artificial Sequence

<220>
<223>  pep5

<400>  5

Thr Glu Asp Glu Leu Gln Asp Lys
1               5


<210>  6
<211>  6
<212>  PRT
<213>  Artificial Sequence

<220>
<223>  pep6

<400>  6

Leu Asn Pro Trp Asp Gln
1               5


<210>  7
<211>  5
<212>  PRT
<213>  Artificial Sequence

<220>
<223>  pep7

<400>  7

Ile Ile Ser Gln Glu
1               5


<210>  8
<211>  6
<212>  PRT
<213>  Artificial Sequence

<220>
<223>  pep8

<400>  8

Ala Asn Glu Glu Glu Tyr
1               5
```

```
<210>    9
<211>    4
<212>    PRT
<213>    Artificial Sequence

<220>
<223>    pep9

<400>    9

Val Ile Pro Tyr
1


<210>    10
<211>    4
<212>    PRT
<213>    Artificial Sequence

<220>
<223>    pep10

<400>    10

Leu Pro Leu Pro
1


<210>    11
<211>    9
<212>    PRT
<213>    Artificial Sequence

<220>
<223>    pep11

<400>    11

Val Ile Glu Ser Pro Pro Glu Ile Asn
1                   5


<210>    12
<211>    4
<212>    PRT
<213>    Artificial Sequence

<220>
<223>    pep12

<400>    12

Phe Pro Gln Tyr
1


<210>    13
<211>    6
<212>    PRT
<213>    Artificial Sequence

<220>
```

```
<223>  pep13

<400>  13

Leu Asn Val Pro Gly Glu
1               5


<210>  14
<211>  8
<212>  PRT
<213>  Artificial Sequence

<220>
<223>  pep14

<400>  14

Ile Val Pro Asn Ser Ala Glu Glu
1               5


<210>  15
<211>  8
<212>  PRT
<213>  Artificial Sequence

<220>
<223>  pep15

<400>  15

Gly Glu Pro Thr Ser Thr Pro Thr
1               5


<210>  16
<211>  9
<212>  PRT
<213>  Artificial Sequence

<220>
<223>  pep16

<400>  16

Lys Phe Gln Ser Glu Glu Gln Gln Gln
1               5
```

**Description**

Technical Field

The present invention relates to a composition for sympathetic nerve activation. The composition can be used as drugs, foods and drinks, or feeds.

Background Art

The autonomic nerve is composed of sympathetic nerve and parasympathetic nerve. These two nervous systems act almost antagonistically on each of internal organs, organs, and tissues to constantly regulate various functions of the living body and maintain homeostasis. Of the autonomic nerves, the sympathetic nerve, which is also called the nerve of struggle and escape, controls various mental and physical active functions by its activation.

Specifically, it is known that activating the sympathetic nerve provides effects such as promotion of energy expenditure, reduction of fatigue, , increase of alertness, drowsiness improvement, improvement of concentration/attention, improvement of physical activity, improvement of mental function, improvement of heat production, increased body temperature, improvement of basal metabolism, promoted sweating, suppression of weight gain, and suppression of body fat accumulation (Patent Literatures 1 to 3).

For example, polyphenols, orotic acid, and caffeine have been reported as components that activate sympathetic nerves (Patent Literatures 4 and 5 and Non-Patent Literature 1) .

Citation List

Patent Literatures

PTL 1: JP-A-2018-87171
PTL 2: JP-A-2012-126683
PTL 3: WO 2017/014120
PTL 4: WO 2018/101231
PTL 5: JP-A-2012-126683

Non-Patent Literature

NPTL: Dulooo AG.et al., Am. J. Clin. Nutr. 1989 49(1) 44-50

Summary of Invention

Technical Problem

An object of the present invention is to provide a technique capable of activating sympathetic nerve. Solution to Problem

As a result of intensive investigations to solve the above problem, the present inventors have found that a peptide having a specific sequence has an excellent sympathetic nerve activating effect, and have completed the present invention.

That is, the present invention is a composition for sympathetic nerve activation (hereinafter, also referred to as "composition of the present invention") comprising one or more peptides selected from the group consisting of peptides consisting of any of amino acid sequences shown below (SEQ ID NOs: 1 to 16) and peptides including the amino acid sequences.

(Amino acid sequences: VAPFPE, HLPLP, WPPF, HQPHQPLPPT, TEDELQDK, LNPWDQ, IISQE, ANEEEY, VIPY, LPLP, VIESPPEIN, FPQY, LNVPGE, IVPNSAEE, GEPTSTPT, KFQSEEQQQ)

In a preferable aspect, the composition of the present invention is used for one or more selected from the group consisting of promotion of energy expenditure, reduction of fatigue, increase of alertness, drowsiness improvement, improvement of concentration/attention, improvement of physical activity, improvement of mental function, improvement of heat production, increased body temperature, improvement of basal metabolism, promoted sweating, suppression of weight gain, and suppression of body fat accumulation.

In a preferable aspect, the composition of the present invention is drugs.

In a preferable aspect, the composition of the present invention is foods and drinks.

Another aspect of the present invention is to use one or more peptides selected from the group consisting of peptides consisting of amino acid sequences shown by any of SEQ ID NOs: 1 to 16 and peptides including the amino acid sequences, in the production of the composition for sympathetic nerve activation.

Another aspect of the present invention is one or more peptides selected from the group consisting of peptides consisting of any of amino acid sequences shown by any of SEQ ID NOs: 1 to 16 and peptides including the amino acid sequences, which are used for sympathetic nerve activation.

Another aspect of the present invention is to use one or more peptides selected from the group consisting of peptides consisting of amino acid sequences shown by any of SEQ ID NOs: 1 to 16 and peptides including the amino acid sequences, for sympathetic nerve activation.

Another aspect of the present invention is a method of activating the sympathetic nerve, including administration of one or more peptides selected from the group consisting of peptides consisting of amino acid sequences shown by any of SEQ ID NOs: 1 to 16 and peptides including the amino acid sequences, to a subject in need of sympathetic nerve activation.

In addition, the present invention includes a milk protein hydrolysate comprising one or more peptides selected from the group consisting of peptides consisting of amino acid sequences shown by any of SEQ ID NOs: 1 to 16 and peptides including the amino acid sequences.

Advantageous Effects of Invention

The present invention provides an excellent composition for sympathetic nerve activation. The composition of the present invention can be used to provide various effects obtained by activating the sympathetic nerve, specifically, promotion of energy expenditure, reduction of fatigue, increase of alertness, drowsiness improvement, improvement of concentration/attention, improvement of physical activity, improvement of mental function, improvement of heat production, increased body temperature, improvement of basal metabolism, promoted sweating, suppression of weight gain, and suppression of body fat accumulation. In addition, the composition of the present invention can be preferable for the aspect of the drug or the food and drink.

Brief Description of Drawings

[Fig. 1] A graph showing the energy expenditure of mice administered with a peptide according to the present invention for 60 minutes after administration.

[Fig. 2] A graph showing the energy expenditure of mice administered with the peptide according to the present invention for 30 minutes after administration.

[Fig. 3] A graph showing the amount of spontaneous exercise for 60 minutes after the end of forced exercise in mice to which a casein hydrolysate including the peptide according to the present invention was administered.

[Fig. 4] A graph showing changes in the sensation before and after the ingestion period of a subject who ingested the casein hydrolysate including the peptide according to the present invention for 2 weeks.

[Fig. 5] A graph showing changes in body composition before and after the ingestion period of a subject who ingested the casein hydrolysate including the peptide according to the present invention for 4 weeks.

[Fig. 6] A graph showing the post-ingestion bodily-sensation effect of a subject who ingested the casein hydrolysate including the peptide according to the present invention for 2 weeks.

Description of Embodiments

The present invention will be specifically described. However, the present invention is not limited to the following embodiments and can be freely changed within the scope of the present invention.

A composition for sympathetic nerve activation according to the present invention comprises, as an active component, one or more peptides selected from the group consisting of peptides consisting of any of amino acid sequences shown below (SEQ ID NOs: 1 to 16) and peptides including the amino acid sequences (hereinafter also referred to as "peptide according to the present invention") .

(Amino acid sequences: VAPFPE, HLPLP, WPPF, HQPHQPLPPT, TEDELQDK, LNPWDQ, IISQE, ANEEEY, VIPY, LPLP, VIESPPEIN, FPQY, LNVPGE, IVPNSAEE, GEPTSTPT, KFQSEEQQQ)

Herein, in the above amino acids, A represents an alanine residue, N an asparagine residue, D an aspartic acid residue, Q a glutamine residue, E a glutamic acid residue, G a glycine residue, H a histidine residue, I an isoleucine residue, L a leucine residue, K a lysine residue, F a phenylalanine residue, P a proline residue, S a threonine residue, W a tryptophan residue, Y a tyrosine residue, and V a valine residue.

The peptide according to the present invention may be included in the form of a salt thereof.

The peptide according to the present invention may be obtained by decomposing a protein (protein hydrolysate) derived from, for example, milk, soybeans, eggs, wheat, barley, rice, potatoes, sweet potatoes, pea, corn, livestock, fish, and seafood. In addition, the peptide according to the present invention may be further isolated and purified from the above decomposed product. In addition, the peptide according to the present invention may be obtained by chemical synthesis or biosynthesis.

In the present invention, the peptide including the amino acid sequences shown by any of SEQ ID NOs: 1 to 16 refers to a peptide obtained by adding any amino acid having one or more peptide residues to the N-terminal and/or C-terminal of the sequence, more specifically, a peptide having 1 to 10 residues, preferably 1 to 5 residues, and more preferably 1 to 3 residues added, and the sequence having a sympathetic nerve activating effect is preferable.

The composition of the present invention has an action of sympathetic nerve activation.

The activation of the sympathetic nerve can be confirmed, for example, by increased energy expenditure, increased oxygen uptake, and increased blood adrenaline level. In addition, the activation of the sympathetic nerve can be confirmed by measuring autonomic nerve activity with well-known electrophysiological methods.

In addition, in the present invention, the "activation" of the sympathetic nerve includes increasing the activity level of the normal sympathetic nerve to increase the amount of activity, recovering the level of sympathetic nerve activity that has decreased due to various causes to increase the amount of sympathetic nerve activity to normal or more, and/or increasing the amount of activity of the sympathetic nerve compared to the parasympathetic nerve.

The activated sympathetic nerve increases energy expenditure. Therefore, the composition of the present invention has an action of promoting the energy expenditure. In general, homeostasis control of human energy balance is maintained within 1% of daily energy intake and expenditure (Takashi Ando, Physical Fitness Science Vol. 67, No. 5, 327-344, (2018)), and energy expenditure increased by more than 1% indicates that energy expenditure has been promoted.

The energy expenditure can be determined by an indirect calorimetry method. The energy expenditure can be calculated by using the following formula according to the method of Tadaishi et al. PLoS One. 2011 6 (12) e28290.

$$\text{Energy expenditure (cal/min)} = 3.91\ VO_2\ (\text{ml/min}) + 1.10\ VCO_2\ (\text{ml/min})$$

In the present invention, energy expenditure is not particularly limited, and refers to those that include energy expenditure by basal metabolism, energy expenditure due to diet-induced heat production, energy expenditure due to non-motor activity heat production caused by daily physical activity, and exercise-induced heat production.

In the present description, exercise refers to not only exercise as a competition, but also activities such as sports, walking, and jogging that are planned and intentionally performed for health, physical fitness, and fun, and physical activity refers to physical activity in daily life, excluding exercise, such as labor, housework, commuting to work/school, and hobbies.

In addition, in the present invention, promotion of energy expenditure includes increasing energy expenditure from a normal energy expenditure level, recovering an energy expenditure level that has decreased due to various causes to increase the energy expenditure to normal or more, and/or further promoting energy expenditure increased after exercise.

In addition, the activated sympathetic nerve promotes the secretion of adrenaline, thereby providing the effects such as increase of alertness, reduction in fatigue, improvement in the feeling of weakness, improvement in drowsiness, improvement in concentration and attention, improvement in physical activity, and improvement in mental function. Herein, the physical activity includes improvement in athletic ability or extension of exercise duration, shortening the required time in competitions and exercises competing for the shortest required time, and suppressing the decrease in reflex ability.

In addition, activating the sympathetic nerve and promoting energy expenditure associated therewith can provide the effects such as improvement in heat production, increased body temperature, improvement in basal metabolism, promotion of sweating, suppression of weight gain, or suppression of body fat accumulation. As a result, the effects of prevention/improvement of body temperature decrease and improvement in peripheral blood flow are expected. Furthermore, there are expected the effects of improvement and prevention of, for example, hypotension due to persistent hypothermia or poor blood circulation in the periphery, decreased visceral function (constipation/diarrhea), immune weakness, poor blood circulation (coldness, stiff shoulders, back pain, arthralgia), sleeping disorder, and hypometabolism (obesity) disease.

Therefore, in a preferable aspect, the composition of the present invention can be used to improve the conditions involving sympathetic nerve activation, specifically, promotion of energy expenditure, reduction of fatigue, increase of alertness, drowsiness improvement, improvement of concentration/attention, improvement of physical activity, improvement of mental function, improvement of heat production, increased body temperature, improvement of basal metabolism, promoted sweating, suppression of weight gain, and suppression of body fat accumulation.

The compositions of the present invention for the conditions or diseases described above can be applied to humans or non-human animals (particularly mammals). In addition, the application may be therapeutic use or non-therapeutic use. The term "non-therapeutic" is a concept that includes no medical practice, that is, no treatment of the human body.

That is, another aspect of the present invention is a method of activating sympathetic nerve, the method including a step of administering the composition for sympathetic nerve activation of the present invention to a subject. Furthermore, the method may be, by sympathetic nerve activation, a method of promoting energy expenditure, a method of reducing fatigue, a method of improving weakness/fatigue, a method of increase of alertness, a method of improving drowsiness, a method of improving concentration/attention, a method of improving physical activity, a method of improving mental function, a method of improving heat production, a method of increasing body temperature, a method of improving basal metabolism, a method of promoting sweating, a method of suppressing weight gain, and a method of suppressing body fat accumulation.

Herein, the term "improvement" means change in the condition for the better, or prevention or delay of deterioration of the condition.

In addition, in the present description, "prevention" means protection or delay of occurrence of a disease/symptom.

The present invention is to use one or more peptides selected from the group consisting of peptides consisting of amino acid sequences shown by any of SEQ ID NOs: 1 to 16 and peptides including the amino acid sequences, in the production of the composition for sympathetic nerve activation.

The present invention is one or more peptides selected from the group consisting of peptides consisting of any of amino acid sequences shown by any of SEQ ID NOs: 1 to 16 and peptides including the amino acid sequences, which are used for sympathetic nerve activation.

The present invention is to use one or more peptides selected from the group consisting of peptides consisting of amino acid sequences shown by any of SEQ ID NOs: 1 to 16 and peptides including the amino acid sequences, for sympathetic nerve activation.

The present invention is a method of activating sympathetic nerve, including administration of one or more peptides selected from the group consisting of peptides consisting of amino acid sequences shown by any of SEQ ID NOs: 1 to 16 and peptides including the amino acid sequences, to a subject in need of sympathetic nerve activation.

The content of the peptide according to the present invention in the composition for sympathetic nerve activation of the present invention can be set to 0.0000001 to 100% by mass, preferably 0.000001 to 20% by mass, more preferably 0. 000005 to 10% by mass, and more preferably 0.000005 to 5% by mass.

In addition, when the peptide according to the present invention is comprised as the aspect of a proteolytic product in the composition for sympathetic nerve activation of the present invention, the content of the proteolytic product can be set to 0.00001 to 50% by mass, preferably 0.0001 to 20% by mass, and more preferably 0.0005 to 10% by mass, with respect to the final composition.

In addition, in the present description, the final composition means a composition when administered to a subject or when ingested by a subject.

The content of the peptide according to the present invention in the final composition can be measured by the following method.

(a) The final composition is diluted and dissolved in a 0.2% formic acid aqueous solution so as to have a concentration of 1.0 mg/mL, and LC/MS analysis is performed under the following measurement condition. Whereas, several solutions of chemically synthesized standard peptides (manufactured by Bio-Synthesis, Inc.) of each peptide sequence of SEQ ID NOs: 1 to 16 are prepared according to the concentration, and LC/MS analysis is performed under the following measurement condition to prepare a calibration curve.

Of the peaks in the analysis of the final composition solution, those whose molecular weight and retention time match the peaks of the standard peptide are identified as the same peptide sequence present in the final composition. The content of each peptide in the final composition is determined by comparing the peak area of the standard peptide with the peak area of the final composition.

(b) LC/MS equipment

Mass spectrometer: TSQ Quantum Discovery MAX (manufactured by Thermo Fisher Scientific Inc.).
High performance liquid chromatograph: Prominence (manufactured by Shimadzu Corporation), column: TSKgel ODS-100V φ2.0 mm x 250 mm, 5.0 μm (manufactured by Tosoh Corporation).

(c) LC/MS measurement condition

Mobile phase A: 0.2% by mass of formic acid aqueous solution
Mobile phase B: 0.2% by mass of formic acid-acetonitrile solution
Sample injection volume: 10 μL, column temperature: 40°C, liquid flow rate: 200 μL/min
Analysis mode: SRM measurement
Product Mass and Parent Mass: Set to m/z for each of the peptides of SEQ ID NOs: 1 to 16.

In the present invention, the dose or intake of the peptide according to the present invention for a subject varies depending on, for example, age and symptoms, and is preferably 0.00001 to 100 mg/kg body weight/day, more preferably 0.0001 to 50 mg/ kg body weight/day, and more preferably 0.0001 to 30 mg/kg body weight/day. In addition, the peptide according to the present invention may be administered or ingested once to three times a day.

The composition of the present invention can preferably be in the aspect of the drug.

Such drugs are used for sympathetic nerve activation and/or prevention, improvement and/or treatment of sympathetic nerve activity reduction, or for improvement of the condition associated with sympathetic nerve activity reduction as described above.

The route of administration of the drug may be oral or parenteral, and oral administration is preferable. In addition, parenteral administration includes intravenous injection, rectal administration, and inhalation. The drug can be appropriately formulated into a desired dosage form according to the administration method. For example, in the case of oral administration, the formulation can include solid dosage forms such as powders, granules, tablets, and capsules; and liquid dosage forms such as solutions, syrups, suspensions, and emulsions. In addition, in the case of parenteral administration, the formulation can include, for example, a suppository and an ointment.

In the formulation, in addition to the peptides according to the present description, components such as excipients, pH adjusters, colorants, and flavoring agents that are typically used in the formulation can be used. In addition, it is possible to use in combination, for example, a known or future drug having the sympathetic nerve activating effect, a lipid metabolism abnormality improving drug, or a hyperlipidemia therapeutic drug.

In addition, formulation can be performed by a known method as appropriate depending on the dosage form. In formulation, a formulation carrier may be blended and formulated as appropriate.

Examples of the excipients include: sugar derivatives such as lactose, sucrose, glucose, mannitol, and sorbitol; starch derivatives such as corn starch, potato starch, α-starch, dextrin, and carboxymethyl starch; cellulose derivatives such as crystalline cellulose, hydroxypropyl cellulose, hydroxypropylmethyl cellulose, carboxymethyl cellulose, and carboxymethyl cellulose calcium; gum arabic; dextran; pullulan; silicate derivatives such as light anhydrous silicic acid, synthetic aluminum silicate, and magnesium metasilicate aluminate; phosphate derivatives such as calcium phosphate; carbonate derivatives such as calcium carbonate; and sulfate derivatives such as calcium sulfate.

In addition to the above excipients, examples of the binder include: gelatin; polyvinylpyrrolidone; and macrogol.

In addition to the above excipients, examples of the disintegrant include chemically modified starch or cellulose derivatives such as croscarmellose sodium, carboxymethyl starch sodium, and crosslinked polyvinylpyrrolidone.

Examples of the lubricant include: talc; stearic acid; metal stearate salts such as calcium stearate and magnesium stearate; colloidal silica; waxes such as veegum and gay wax; boric acid; glycol; carboxylic acids such as fumaric acid and adipic acid; sodium carboxylic acid salts such as sodium benzoate; sulfates such as sodium sulfate; leucine; lauryl sulfates such as sodium lauryl sulfate and magnesium lauryl sulfate; silicic acids such as anhydrous silicic acid and silicic acid hydrate; and starch derivatives.

Examples of the stabilizer include: paraoxybenzoic acid esters such as methylparaben and propylparaben; alcohols such as chlorobutanol, benzyl alcohol, and phenylethyl alcohol; benzalconium chloride; acetic anhydride; and sorbic acid.

Examples of the flavoring agent include sweeteners, acidulants, and flavors.

Examples of the carrier used in the case of a liquid preparation for oral administration include a solvent such as water.

The timing of ingesting the drug of the present invention is not particularly limited, and may be before meals, after meals, between meals, during exercise, or at rest.

The composition of the present invention can preferably be in the aspect of the food and drink.

The food and drink can be provided and sold as the food and drink that are labeled the use for sympathetic nerve activation or the use for improving the condition with which the activity of the sympathetic nerve is involved.

As long as such "labeling" act includes all acts for informing the consumer of the above use and the label describes the expression such that the above use can be recalled or analogized, all of the labels correspond to the "labeling" act of the present invention regardless of the purpose of the label, the content of the label, and the object or medium to be labeled.

In addition, the "labeling" preferably includes an expression that allows the consumer to directly recognize the above use. Specific examples thereof include: the acts of transferring, delivering, or displaying and importing for transferring or delivering a food-and-drink related product or a packaging thereof with the above use described; and the acts of describing the above use in advertisements, price lists, or transaction documents and then displaying or distributing, or describing the above use in the information including these contents and then providing by an electromagnetic method (for example, Internet).

Whereas, the label is preferably approved by, for example, the government as the content of the label (for example, a label approved based on various systems established by the government and expressed in the aspect based on such approval). In addition, such a label content is preferably attached to packaging, containers, catalogs, pamphlets, promotional materials such as POP at sales sites, and other documents.

In addition, "labeling" includes labels such as health foods, functional foods, enteric nutritional foods, special purpose foods, health functional foods, specified health foods, nutritional functional foods, functional display foods, and non-medicinal products. Of these, particularly, "labeling" includes the label approved by the Consumer Affairs Agency, for example, a label approved by a system related to specified health foods, nutritional functional foods, or functional display foods, or a system similar to these. Specific examples thereof can include a label as specified health foods, a label as specified health foods with conditions, a label describing that the structure and function of the body are affected, a label of reducing the risk of illness, and a label of the functionality based on scientific evidence, and more specifically, typical examples thereof are a label as specified health foods stipulated in the Cabinet Office Ordinance (Cabinet Office Ordinance No. 57 of August 31, Heisei 21) regarding permission for special use labeling prescribed in the Health Promotion Law (particularly the label for health use) and a label similar to this.

Examples of such a label include, for those who are exercising and/or on a diet, "those who want to improve energy expenditure", "those who want to improve metabolism", "those who want to reduce body fat", "those who are feeling tired", "to make your body less tired", "improvement of weakness/malaise or when feelings and body feel tired", "to improve drowsiness", "for those who want to improve their concentration and attention", "to improve physical activity", "to improve mental function", "for a constitution that makes it easy to sweat", "don't gain too much weight", "for those who are concerned about visceral fat", "those who are concerned about a decrease in basal metabolism", "those who want to warm their bodies", and "those who have poor circulation".

In addition, the peptide according to the present description or the proteolytic product comprising the peptide of the present invention may be contained in the food and drink to be processed as one aspect of the food and drink of the present invention.

Regardless of the form such as liquid, paste, solid, and powder, examples of the food and drink include, in addition to tablet confectionery, liquid foods, feeds including for pets, wheat flour products, instant foods, processed agricultural products, processed marine products, processed livestock products, milk and dairy products, oils and fats, basic seasonings, complex seasonings and foods, frozen foods, confectionery, beverages, and commercial foods other than these.

Examples of the wheat flour product include bread, macaroni, spaghetti, noodles, cake mix, fried chicken flour, and bread crumbs.

Examples of the instant foods include instant noodles, cup noodles, retort and cooked foods, canned foods, microwave foods, instant soups and stews, instant miso soups and soups, canned soups, freeze-dried foods, and other instant foods.

Examples of the processed agricultural products include canned agricultural products, canned fruits, jams and marmalades, pickles, boiled beans, dried agricultural products, and cereals (processed grain products).

Examples of the processed marine products include canned marine products, fish hams and sausages, fish paste products, fishery delicacies, and tsukudani.

Examples of the processed livestock products include canned livestock and pastes, livestock ham and sausage.

Examples of the milk and dairy products include processed milk, milk drinks, yogurts, lactic acid bacteria drinks, cheese, ice creams, formula milk powders, creams, and other dairy products.

Examples of the fats and oils include butter, margarines, and vegetable oils.

Examples of the basic seasoning include soy sauce, miso, sauces, processed tomato seasonings, mirin, and vinegar.

Examples of the complex seasonings and foods include cooking mixes, curry ingredients, sauces, dressings, noodle soups, spices, and other complex seasonings.

Examples of the frozen food include raw material frozen food, semi-cooked frozen food, and cooked frozen food.

Examples of the confectionery include caramel, candy, chewing gum, chocolate, cookies, biscuits, cakes, pies, snacks, crackers, Japanese confectionery, rice confectionery, bean confectionery, dessert confectionery, and other confectionery.

Examples of the beverages include carbonated drinks, natural juices, juice drinks, soft drinks with fruit juice, pulp drinks, fruit drinks with fruit grains, vegetable drinks, soy milk, soy milk drinks, coffee drinks, tea drinks, powdered drinks, concentrated beverages, sports beverages, energy drinks, alcoholic beverages, and other favorite beverages.

Examples of commercial foods other than the above include baby food, sprinkle, and ochadsuke seaweed.

Examples

Hereinafter, the present invention will be described in more detail using examples, but the present invention is not limited to these examples.

<1> Confirmation 1 of sympathetic nerve activating effect by the peptide according to the present invention

Dietary intake increases energy expenditure (meal-induced heat production), and it has been reported that heat production has two stages: the initial stage (up to about 45 minutes from ingestion) in which the heat production occurs mainly through sympathetic nerve activity and the second stage (after 45 minutes from ingestion) in which the heat production occurs mainly during the process of digestion, absorption, oxidation, and storage of nutrient (P. Diamond et al., Am. J. Physiol., 1987, Jun; 252 (6 Pt 1): E719-26). In the following experiments, the energy expenditure for 30 minutes or 60 minutes from the administration of the test sample, which is considered to correspond to the heat production in the initial stage, was measured and used as an index of sympathetic nerve activity.

Male C57BL/6J mice (n = 9) were fasted for 1 hour, and then 1 $\mu$g/g body weight of peptides (synthetic peptides of SEQ ID NOs: 1 to 16) or the same amount of water was orally administered. Exhaled breath was continuously collected for 60 minutes after administration, and energy expenditure was determined by indirect calorimetry from the oxygen concentration and carbon dioxide concentration in the exhaled breath. The energy expenditure was calculated by using the following formula according to the method of Tadaishi et al. (PLoS One. 2011 6 (12) e28290).

$$\texttt{Energy expenditure (cal/min) = 3.91 VO}_2 \texttt{ (ml/min) + 1.10}$$
$$\texttt{VCO}_2 \texttt{ (ml/min)}$$

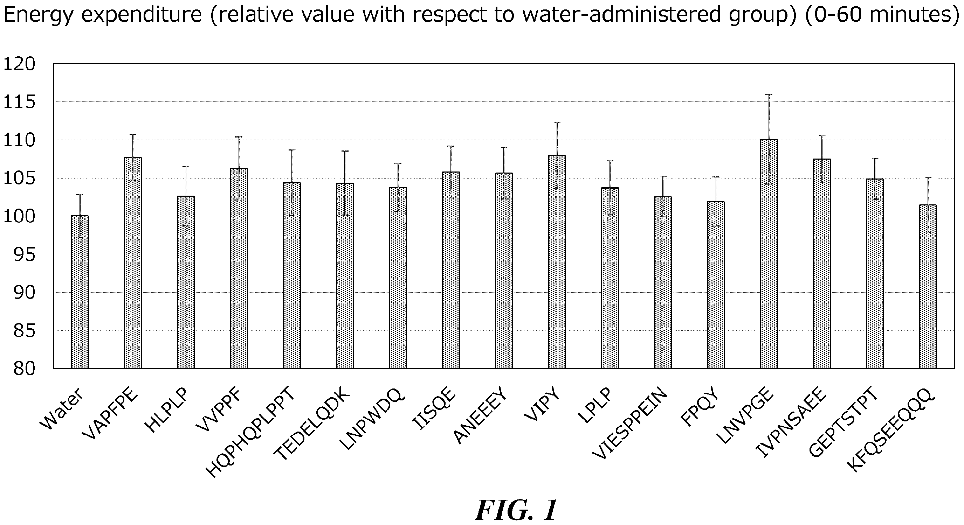

The relative value of energy expenditure (AUC) for 60 minutes after administration with respect to the water-administered group is shown in Fig. 1. Energy expenditure increased in each peptide-administered group as compared with the water-administered group. These results have found that the peptides of SEQ ID NOs: 1 to 16 have the sympathetic nerve activating effect.

<2> Confirmation 2 of sympathetic nerve activating effect by the peptide according to the present invention

The same test as in <1> was performed by using the peptides of SEQ ID NOs: 1, 7, 9 and 13 to 15. Each group was set to n = 8, and a crossover test was performed.

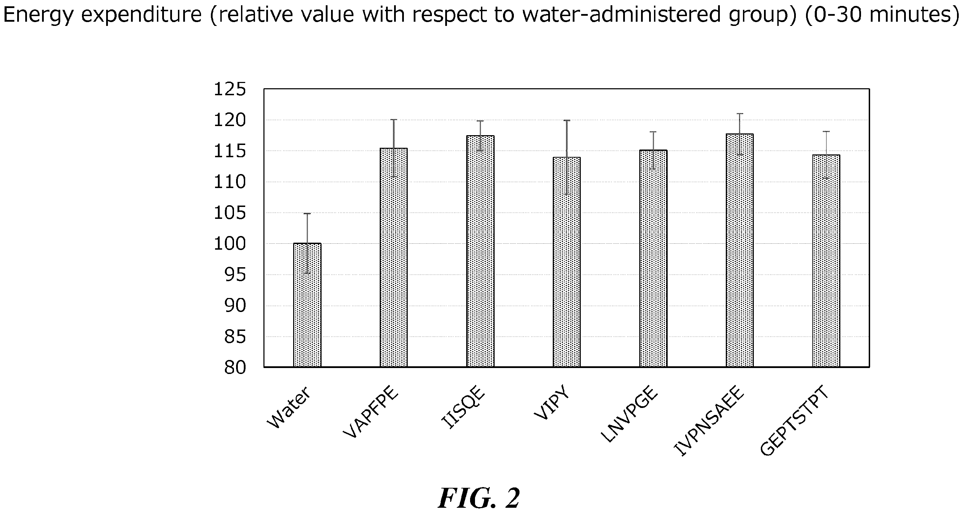

The relative value of energy expenditure (AUC) for 30 minutes after administration with respect to the water-administered group is shown in Fig. 2. Energy expenditure increased in each peptide-administered group as compared with the water-administered group. These results have found that the peptides of SEQ ID NOs: 1, 7, 9 and 13 to 15 have the sympathetic nerve activating effect.

<3> Acquisition of casein hydrolysate comprising the peptide according to the present invention

Casein (manufactured by Fonterra Co-operative Group Limited) was dissolved in water to prepare a casein aqueous solution having a concentration of about 10% by mass. The casein aqueous solution was heat-sterilized by a conventional method, and then the temperature and pH were adjusted to the optimum ranges, and a commercially available proteolytic enzyme was added to initiate a hydrolysis reaction. When the decomposition rate of casein reached 20 to 30%, the enzyme was inactivated by heating to stop the enzyme reaction, and the hydrolysate was obtained by concentrating and powdering by a conventional method. In addition, it was confirmed that the obtained hydrolysate comprised two or more of peptides of SEQ ID NOs: 1 to 16 in an amount of 0.0005 to 1.5% by mass with respect to the total hydrolysate.

<4> Confirmation of the effect of reducing the feeling of fatigue by the casein hydrolysate comprising the peptide according to the present invention

Based on the fact that the amount of spontaneous exercise in the rotating cage after the forced exercise load of the mouse is known to be an index of fatigue (Y. Takahashi, et al., J. Phys. Fitness Sports Med., 2 ( 3), 373-379 (2013)), the fatigue-reducing effect of the peptide was confirmed by the following experiments.

Male BALB/c mice were divided into two groups, a water-administered group and a casein hydrolysate-administered group. The one prepared in <3> was used as the casein hydrolysate. After fasting for 4 hours and 30 minutes, 1.0 mg/g body weight of the casein hydrolysate or the same amount of water was orally administered. Thirty minutes after oral administration, the mice were forcibly exercised for 30 minutes at a speed of 20 m/min with a treadmill device (Muromachi Kikai Co., Ltd.). After the forced exercise was completed, the amount of spontaneous exercise (rotation speed/minute) for 60 minutes was measured from the rotation speed of the rotating cage provided in the breeding cage. In this experiment, each group was set to n = 8, and a crossover test was performed.

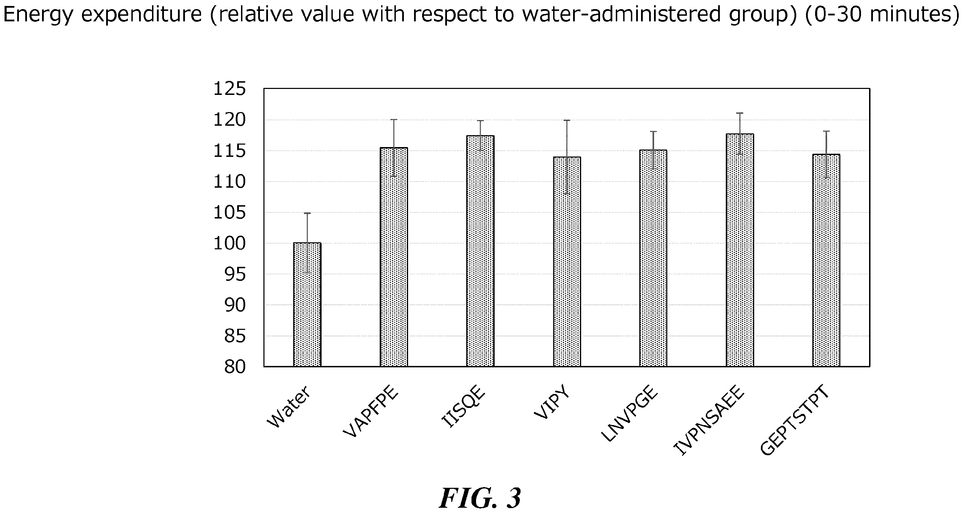

The amount of spontaneous exercise for 60 minutes after forced exercise is shown in Fig. 3. Compared with the water-administered group, the casein hydrolysate group showed a higher amount of spontaneous exercise. This result has found that the casein hydrolysate comprising the peptides of SEQ ID NOs: 1 to 16 reduces the feeling of fatigue caused by exercise. In addition, spontaneous exercise by the rotating cage shows motivation to exercise, and therefore is found to have the effects of improving weakness/fatigue, increase of alertness, improving physical activity, and improving mental function.

<5> Confirmation of bodily sensation effect and effect on body composition by ingestion of peptide according to the present invention

Changes in bodily sensation when the casein hydrolysate comprising the peptide according to the present invention was continuously ingested for 2 weeks and changes in body composition when continuously ingested for 4 weeks were investigated.

Specifically, for subjects of women with continuous exercise habits at least three times a week, the powdered beverage comprising the casein hydrolysate prepared in <3> was continuously ingested for 2 weeks (5000 mg/dose of casein hydrolysate) or 4 weeks (10000 mg/dose of casein hydrolysate). The subjects were instructed to dissolve and ingest the powdered beverage in about 70 ml of water per 1000 mg of casein hydrolysate. The exercise content and exercise time during the ingestion period were the same as before the start of ingestion.

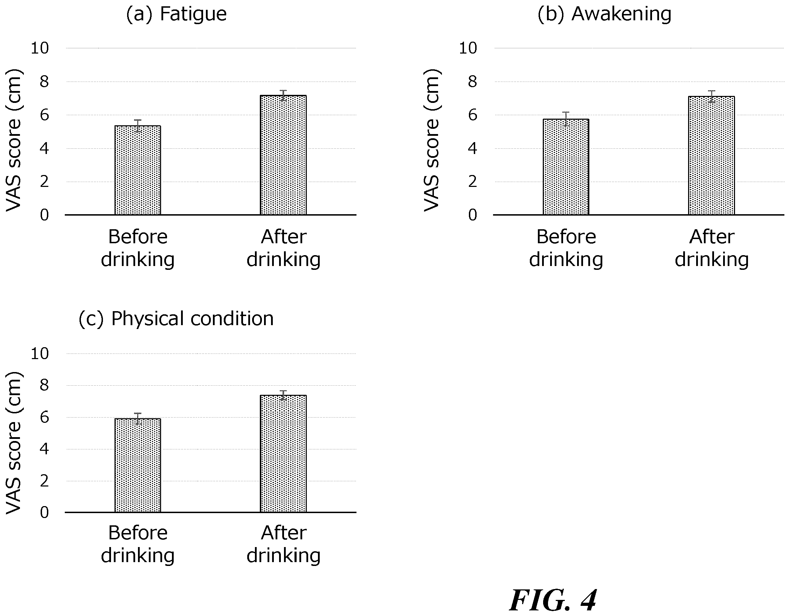

The subjects (38 subjects) who ingested continuously for 2 weeks were asked to evaluate three items of fatigue, awakening, and physical condition before and 2 weeks after the start of ingestion by the Visual Analog Scale (VAS). Fig. 4 shows the result of the change in bodily sensation. The VAS score of fatigue, awakening, and physical condition improved as compared with before ingestion. This result has shown that the casein hydrolysate comprising the peptides of SEQ ID NOs: 1 to 16 has the effect of reducing fatigue, the effect of improving weakness/fatigue, the effect of increase of alertness, and the effect of improving drowsiness.

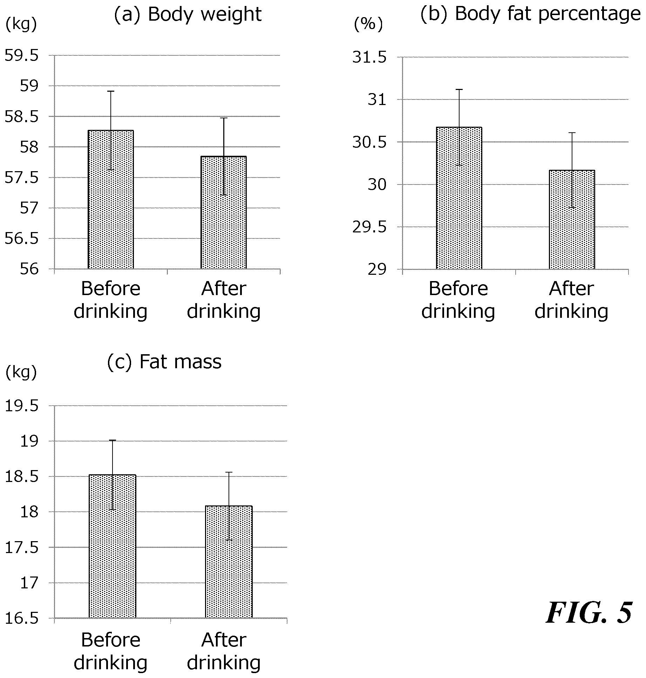

The body weight, body fat percentage, and fat mass of the subjects (257 subjects) who continuously ingested for 4 weeks were measured before the start of ingestion and 4 weeks after the start of ingestion. Fig. 5 shows the results of changes in body composition. The body weight, body fat percentage, and fat mass decreased as compared with those before ingestion, and this has shown the effect of suppressing weight gain and the effect of suppressing body fat accumulation.

<6> Investigation on the bodily sensation effect of peptide ingestion according to the present invention

The subjects of 35 adult women were asked to continuously ingest a powdered beverage mainly comprising casein hydrolysate adjusted in <3> for 2 weeks (2500 mg/dose of casein hydrolysate), and the effect of the bodily sensation after ingestion was investigated. The subjects were instructed to dissolve and ingest the powdered beverage in about 100 to 250 ml of water.

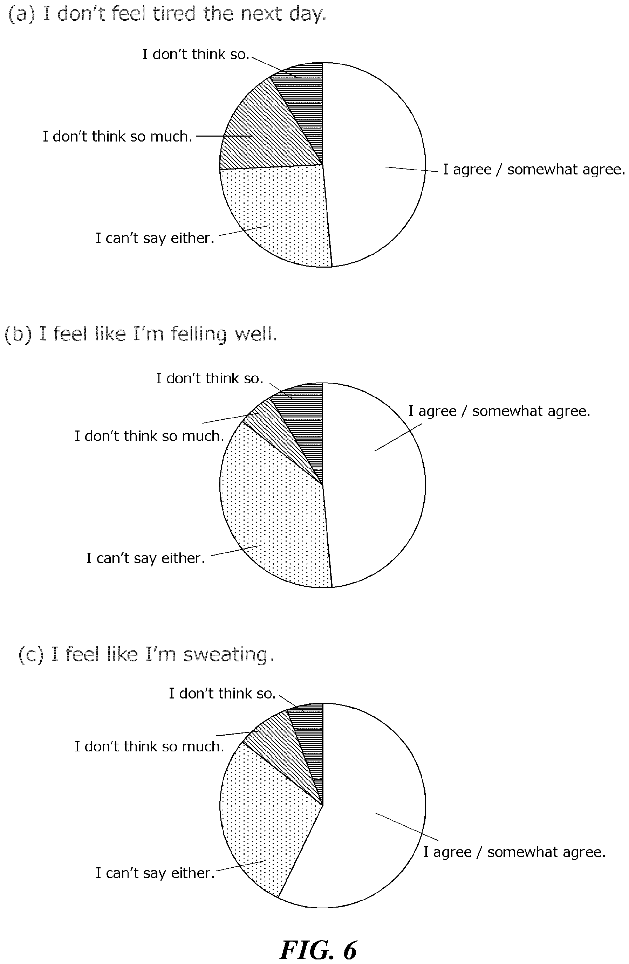

The results are shown in Fig. 6. In response to the questions "I don't feel tired the next day," "I feel like I'm feeling well," and "I feel like I'm sweating", 49%, 49%, and 57% of the subjects answered "I agree / somewhat agree", respectively.

The subjectivity of fatigue and physical condition was improved, and this has shown that the casein hydrolysate comprising the peptides of SEQ ID NOs: 1 to 16 has the effect of reducing fatigue and the effect of improving weakness/fatigue. In addition, fatigue and physical condition are affected by mental condition, and this has shown the effect of improving mental function.

Furthermore, many subjects experienced sweating, and this has shown the sweating effect and the effect of improving body temperature and heat production, which are the background of sweating.

**Claims**

1. A composition for sympathetic nerve activation, comprising:
   one or more peptides selected from the group consisting of peptides consisting of an amino acid sequence shown by any of SEQ ID NOs: 1 to 16 and peptides including the amino acid sequences.

2. The composition according to claims 1, used for one or more selected from the group consisting of promotion of energy expenditure, reduction of fatigue, increase of alertness, drowsiness improvement, improvement of concentration/attention, improvement of physical activity, improvement of mental function, improvement of heat production, increased body temperature, improvement of basal metabolism, promoted sweating, suppression of weight gain, and suppression of body fat accumulation.

3. The composition according to claims 1 or 2, being a drug.

4. The composition according to claims 1 or 2, being a food and drink.

5. Use of one or more peptides selected from the group consisting of peptides consisting of an amino acid sequence shown by any of SEQ ID NOs: 1 to 16 and peptides including the amino acid sequences, in production of a composition for sympathetic nerve activation.

6. One or more peptides selected from the group consisting of peptides consisting of any of amino acid sequences shown by any of SEQ ID NOs: 1 to 16 and peptides including the amino acid sequences, the peptide used for sympathetic nerve activation.

7. Use of one or more peptides selected from the group consisting of peptides consisting of amino acid sequences shown by any of SEQ ID NOs: 1 to 16 and peptides including the amino acid sequences, for sympathetic nerve activation.

8. A method of activating sympathetic nerve, comprising administration of one or more peptides selected from the group consisting of peptides consisting of amino acid sequences shown by any of SEQ ID NOs: 1 to 16 and peptides including the amino acid sequences, to a subject in need of sympathetic nerve activation.

9. A milk protein hydrolysate, comprising:
   one or more peptides selected from the group consisting of peptides consisting of amino acid sequences shown by any of SEQ ID NOs: 1 to 16 and peptides including the amino acid sequences.

Energy expenditure (relative value with respect to water-administered group) (0-60 minutes)

*FIG. 1*

Energy expenditure (relative value with respect to water-administered group) (0-30 minutes)

*FIG. 2*

EP 4 035 671 A1

Energy expenditure (relative value with respect to water-administered group) (0-30 minutes)

*FIG. 3*

FIG. 4

(a) Body weight
(b) Body fat percentage
(c) Fat mass

FIG. 5

EP 4 035 671 A1

(a) I don't feel tired the next day.

I don't think so.

I don't think so much.

I agree / somewhat agree.

I can't say either.

(b) I feel like I'm felling well.

I don't think so.

I don't think so much.

I agree / somewhat agree.

I can't say either.

(c) I feel like I'm sweating.

I don't think so.

I don't think so much.

I can't say either.

I agree / somewhat agree.

*FIG. 6*

## INTERNATIONAL SEARCH REPORT

International application No.

PCT/JP2020/029893

### A. CLASSIFICATION OF SUBJECT MATTER

Int.Cl. A61K38/17(2006.01)i, A61K35/20(2006.01)i, A61K38/07(2006.01)i, A61P3/00(2006.01)i, A61P3/04(2006.01)i, A61P25/02(2006.01)i, A61P25/26(2006.01)i, C07K5/103(2006.01)i, C07K5/107(2006.01)i, C07K14/47(2006.01)i
FI: A61K38/17ZNA, A61K35/20, A61K38/07, A61P3/00, A61P3/04, A61P25/02104, A61P25/26, C07K5/103, C07K5/107, C07K14/47

According to International Patent Classification (IPC) or to both national classification and IPC

### B. FIELDS SEARCHED

Minimum documentation searched (classification system followed by classification symbols)
Int.Cl. A61K38/17, A61K35/20, A61K38/07, A61P3/00, A61P3/04, A61P25/02, A61P25/26, C07K5/103, C07K5/107, C07K14/47

Documentation searched other than minimum documentation to the extent that such documents are included in the fields searched
| | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)
JSTPlus/JMEDPlus/JST7580(JDreamIII), CAplus/MEDLINE/EMBASE/BIOSIS(STN), PubMed, 医中誌 WEB(Ichushi WEB)

### C. DOCUMENTS CONSIDERED TO BE RELEVANT

| Category* | Citation of document, with indication, where appropriate, of the relevant passages | Relevant to claim No. |
|---|---|---|
| X<br>Y | WO 2019/058609 A1 (MORINAGA MILK INDUSTRY CO., LTD.) 28.03.2019 (2019-03-28), claims 1, 2, 4, 6, 8–11, 13, 15, 17, 19–23, paragraphs [0066], [0072]–[0074], fig. 1–3 | 1–9<br>1–8 |
| X | JP 2005-239579 A (AJINOMOTO CO., INC.) 08.09.2005 (2005-09-08), claims 1–4, comparative example 1, examples 1–5, fig. 1–5 | 1–9 |
| X<br>Y | JP 2016-65038 A (MORINAGA MILK INDUSTRY CO., LTD.) 28.04.2016 (2016-04-28), paragraph [0036], production examples 2, 3, tables 2, 3 | 6, 9<br>1–8 |

☒ Further documents are listed in the continuation of Box C.　　☒ See patent family annex.

| | | |
|---|---|---|
| * | Special categories of cited documents: | |
| "A" | document defining the general state of the art which is not considered to be of particular relevance | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 16.09.2020 | 06.10.2020 |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

**INTERNATIONAL SEARCH REPORT**

International application No.

PCT/JP2020/029893

C (Continuation).   DOCUMENTS CONSIDERED TO BE RELEVANT

| Category* | Citation of document, with indication, where appropriate, of the relevant passages | Relevant to claim No. |
|---|---|---|
| X<br>Y | JUILLARD, V. et al., The extracellular PI-type proteinase ox Lactococcus lactis hydrolyzes beta-casein into more than one hundred different oligopeptides, Journal of Bacteriology, 1995, vol. 177, no. 12, pp. 3472-3478, doi:10.1128/jb.177.12.3472-3478.1995, ISSN 0021-9193, particularly, p. 3472, abstract, fig. 4 | 6, 9<br>1-8 |
| X<br>Y | TU, M. et al., Bioactive hydrolysates from casein: generation, identification, and in silico toxicity and allergenicity prediction of peptides, Journal of the Science of Food and Agriculture, 2018, vol. 98, no. 9, pp. 3416-3426, doi: 10.1002/jsfa.8854, ISSN 0022-5142, particularly, p. 3416, "abstract", table 1 (pp. 3420-3422), fig. 4 | 6, 9<br>1-8 |
| Y | 箕越 靖彦，エネルギー代謝に及ぼす視床下部－交感神経系の調節作用，肥満研究, 1999, vol. 5, no. 3, pp. 169-176, ISSN 1343-229X, particularly, p. 169, left column, second paragraph to p. 171, right column, first paragraph, fig. 1-3, (MINAKOSHI, Yasuhiko, Journal of Japan Society for the Study of Obesity), non-official translation (Regulatory role of hypothalamus-sympathetic nervous system in energy metabolism) | 1-8 |
| Y | 奥村 宣明 ほか，生体エネルギー消費系と調節機構 自律神経系による代謝調節，日本臨牀, 2003, vol. 61, extra edition no. 6, pp. 259-264, ISSN 0047-1852, particularly, pp. 260, 261, 「2．自律神経とエネルギー消費」, (OKUMURA, Nobuaki et al., Japanese Journal of Clinical Medicine), non-official translation (Bioenergy consumption system and regulatory mechanism Metabolic regulation by the autonomic nervous system, "2. Autonomic nerves and energy consumption") | 1-8 |
| A | MATSUNAGA, Y. et al., Effects of glucose with casein peptide supplementation on post-exercise muscle glycogen resynthesis in C57BL/6J Mice, Nutrients, 2018, vol. 10, no. 6, 753, doi:10.3390/nul0060753, ISSN 2072-6643, entire text, all drawings | 1-9 |
| A | LILLEFOSSE, H. H. et al., Hydrolyzed casein reduces diet-induced obesity in male C57BL/6J mice, The Journal of Nutrition, 2013, vol. 143, no. 9, pp. 1367-1375, doi:10.3945/jn.112.170415, ISSN 0022-3166, entire text, all drawings | 1-9 |

Form PCT/ISA/210 (second sheet) (January 2015)

INTERNATIONAL SEARCH REPORT
Information on patent family members

| International application No. |
| --- |
| PCT/JP2020/029893 |

```
WO 2019/058609 A1  28.03.2019   (Family: none)

JP 2005-239579 A   08.09.2005   US 2008/0009440 A1
                                claims 1-4, comparative example 1,
                                examples 1-5, fig. 1-5
                                WO 2005/079826 A1
                                EP 1721616 A1

JP 2016-65038 A    28.04.2016   JP 2018-65803 A
```

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018087171 A
- JP 2012126683 A
- WO 2017014120 A
- WO 2018101231 A

**Non-patent literature cited in the description**

- **DULOOO AG.** *Am. J. Clin. Nutr.,* 1989, vol. 49 (1), 44-50
- **TAKASHI ANDO.** *Physical Fitness Science,* 2018, vol. 67 (5), 327-344
- **P. DIAMOND et al.** *Am. J. Physiol.,* June 1987, vol. 252 (6), E719-26
- **TADAISHI et al.** *PLoS One,* 2011, vol. 6 (12), e28290
- **Y. TAKAHASHI et al.** *J. Phys. Fitness Sports Med.,* 2013, vol. 2 (3), 373-379